# EUROPEAN PATENT APPLICATION

(11) **EP 3 493 214 A1**
(43) Date of publication of application: **05.06.2019**
(21) Application number: 18207341.1
(22) Date of filing: 20.11.2018
(51) Int. Cl.: G16H 30/20, G16H 40/63, G16H 40/67

(54) **RADIATION IMAGING SYSTEM, RADIATION IMAGING METHOD, RADIATION IMAGING APPARATUS, AND STORAGE MEDIUM**

(30) Priority: 29.11.2017 JP 2017229366
(71) Applicant: CANON KABUSHIKI KAISHA, Ohta-ku Tokyo 146-8501 (JP)
(72) Inventor: HIKOSAKA, Manami, Tokyo, 146-8501 (JP)
(74) Representative: TBK

(57) **Abstract**

A radiation imaging system includes a plurality of radiation imaging apparatuses configured to perform radiation imaging. The radiation imaging apparatus comprises: protocol storage means configured to store an imaging protocol which includes characteristic information for controlling the radiation imaging; association means configured to associate characteristic information of another imaging protocol of another radiation imaging apparatus with the characteristic information of the stored imaging protocol; and control means configured to control, based on the association, registration of the characteristic information of the other imaging protocol in the protocol storage means .

## Description

### BACKGROUND OF THE INVENTION

### Field of the Invention

The present invention relates to a radiation imaging system that performs imaging by using radiation, a radiation imaging method, a radiation imaging apparatus, and a storage medium.

### Description of the Related Art

In radiation imaging performed in the medical field, imaging is performed based on an imaging protocol which includes information related to at least one of an imaging part, an imaging table, image processing, an imaging condition, and the like required for the imaging.

In Japanese Patent Laid-Open No. 2016-22096, there is proposed a technique in which imaging protocols that are set in radiation imaging apparatuses, which are installed in a plurality of imaging rooms, are shared between the radiation imaging apparatuses, and an imaging protocol to be shared is used to perform imaging of a subject.

However, if the imaging protocol is shared uniformly, sensor characteristics such as the calibration information of a sensor to be used in the imaging will also be shared, and it may lead to a state in which an unexpected image is obtained because correction processing that is unsuitable for the sensor to be used for imaging is performed.

Therefore, in consideration of the above-described related art, the present invention provides a radiation imaging technique that can control registration, between a plurality of radiation imaging apparatuses that perform imaging protocol sharing, based on the association between the characteristic information of a stored imaging protocol and the characteristic information of another imaging protocol of another radiation imaging apparatus.

### SUMMARY OF THE INVENTION

The present invention in its first aspect provides a radiation imaging system as specified in claims 1 to 18.

The present invention in its second aspect provides a radiation imaging method as specified in claim 19.

The present invention in its third aspect provides a radiation imaging apparatus as specified in claim 20.

The present invention in its fourth aspect provides a storage medium as specified in claim 21.

According to the present invention, it is possible to control registration, between a plurality of radiation imaging apparatuses that perform imaging protocol sharing, based on the association of the characteristic information of a stored imaging protocol and the characteristic information of another imaging protocol of another radiation imaging apparatus.

Further features of the present invention will become apparent from the following description of exemplary embodiments with reference to the attached drawings.

### BRIEF DESCRIPTION OF THE DRAWINGS

Fig. 1 is a block diagram showing the system arrangement of a radiation imaging system according to the first embodiment;
Fig. 2 is a block diagram showing the functional arrangement of the radiation imaging system;
Fig. 3 is a block diagram showing an example of imaging protocols and an imaging protocol information group;
Fig. 4 is a flowchart for explaining a processing procedure in which a protocol output unit of an output-side radiation imaging apparatus stores a protocol information group in a data storage unit;
Fig. 5 is a flowchart for explaining a processing procedure in which a control unit 202e of a reading-side radiation imaging apparatus stores the protocol information group in the protocol storage unit;
Fig. 6 is a flowchart for explaining a more specific processing procedure of imaging protocol reflection processing;
Fig. 7 is a view showing an example of a GUI for designating an association between pieces of imaging table information;
Fig. 8 is a block diagram showing the functional arrangement of a radiation imaging system;
Fig. 9 is a flowchart for explaining a processing procedure in which a protocol output unit of an output-side radiation imaging apparatus stores a protocol information group in a data storage unit of a reading-side radiation imaging apparatus;
Fig. 10 is a flowchart for explaining a processing procedure in which a control unit 802e of the reading-side radiation imaging apparatus stores the protocol information group in the protocol storage unit;
Fig. 11 is a block diagram showing the system arrangement of a radiation imaging system according to the third embodiment;
Fig. 12 is a block diagram showing the functional arrangement of the radiation imaging system;
Fig. 13 is a flowchart for explaining a processing procedure in which a protocol output unit of an output-side radiation imaging apparatus stores a protocol information group in a shared server;
Fig. 14 is a flowchart for explaining a processing procedure in which a control unit 1202e of a reading-side radiation imaging apparatus stores the protocol information group in a protocol storage unit of the reading-side radiation imaging apparatus;
Fig. 15 is a flowchart for explaining a processing procedure in which a control unit 202e of a reading-side radiation imaging apparatus selects a registration target on an imaging protocol basis and stores a protocol information group in a protocol storage unit;
Fig. 16 is a flowchart for explaining the procedure of imaging protocol reflection processing;
Fig. 17 is a view showing an example of a GUI for selecting an imaging protocol;
Fig. 18 is a flowchart for explaining a processing procedure in which an output-side radiation imaging apparatus selects an imaging protocol to be output on an imaging protocol basis and stores a protocol information group which includes the selected imaging protocol in a data storage unit;
Fig. 19 is a view showing an example of a GUI for selecting the imaging protocol;
Fig. 20 is a flowchart for explaining a more specific processing procedure of imaging protocol reflection processing;
Fig. 21 is a flowchart for explaining a processing procedure in which characteristic information to be additionally registered is filtered in a reading-side radiation imaging apparatus; and
Fig. 22 is a view showing an example of a GUI to setting characteristic information to be shared.

### DESCRIPTION OF THE EMBODIMENTS

Embodiments of the present invention will be exemplarily described in detail below with reference to the accompanying drawings. Note that the constituent elements described in the embodiments are merely examples. The technical scope of the present invention is determined by the scope of claims and is not limited by the following individual embodiments.

### [First Embodiment]

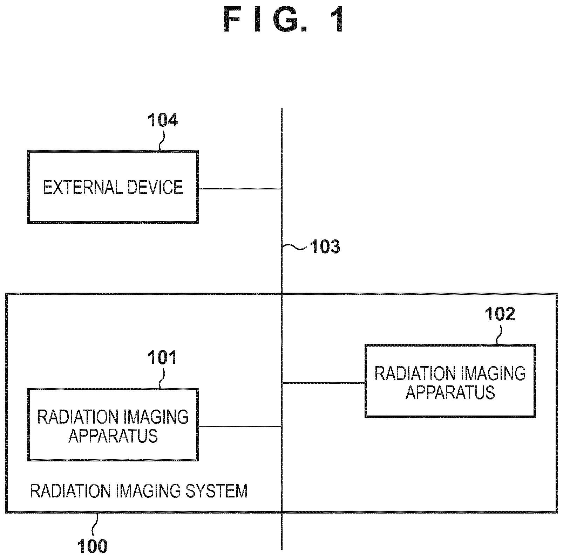

Fig. 1 is a block diagram showing the system arrangement of a radiation imaging system 100 according to the first embodiment of the present invention. The radiation imaging system 100 includes a plurality of radiation imaging apparatuses that perform radiation imaging. For example, the radiation imaging system 100 includes at least one output-side radiation imaging apparatus 101 that outputs imaging information such as an imaging protocol and the like and at least one reading-side radiation imaging apparatus 102 that reads the imaging information such as the imaging protocol and the like. The plurality of radiation imaging apparatuses 101 and 102 are connected to a network 103. The radiation imaging system 100 is connected to an external device 104 such as RIS (Radiology Information Systems) via the network 103. The respective functions of the output-side radiation imaging apparatus 101 and the reading-side radiation imaging apparatus 102 are not fixed, and whether a radiation imaging apparatus is to be an output apparatus or a reading apparatus can be switched by user setting.

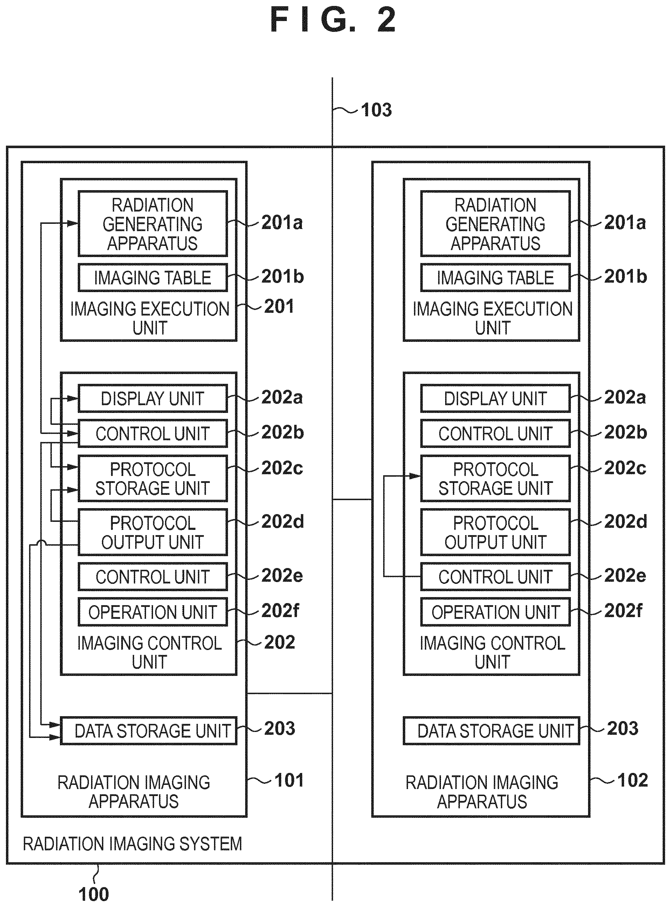

Fig. 2 is a block diagram showing the functional arrangement of the radiation imaging system 100. The radiation imaging apparatuses 101 and 102 each include an imaging execution unit 201, an imaging control unit 202, and a data storage unit 203.

The imaging execution unit 201 includes a radiation generating apparatus 201a that generates radiation and an imaging table 201b. The imaging control unit 202 includes a display unit 202a, a control unit 202b that functions as a first control unit, a protocol storage unit 202c, a protocol output unit 202d, a control unit 202e that functions as a second control unit, and an operation unit 202f. The operations of the output-side radiation imaging apparatus 101 and the reading-side radiation imaging apparatus 102 will be described in detail below.

### (Operation of Output-Side Radiation Imaging Apparatus 101)

In the output-side radiation imaging apparatus 101 (to be also referred to as an output apparatus hereinafter), the control unit 202b controls the radiation generating apparatus 201a and the imaging table 201b by obtaining each imaging protocol, which is required for imaging, from the protocol storage unit 202c storing the imaging protocol that includes characteristic information for controlling a radiation imaging operation. The radiation generating apparatus 201a receives the imaging conditions from the control unit 202b and performs radiation irradiation. The control unit 202b performs control to enable/disable the imaging operation of a detector (sensor) included in the imaging table 201b, and the detector detects the radiation received from the radiation generating apparatus 201a to generate an image and subsequently transmits the generated image to the control unit 202b. The display unit 202a displays an image received by the control unit 202b and displays a GUI for accepting an operation from the operation unit 202f.

The control unit 202b stores the received radiation image in the data storage unit 203. In addition, the protocol output unit 202d obtains a protocol information group that has been set and registered via the protocol storage unit 202c, and stores the obtained protocol information group in the data storage unit 203. The data storage unit 203 stores all of the imaging protocols stored in the protocol storage unit 202c to exchange imaging protocols between the plurality of radiation imaging apparatuses 101 and 102.

### (Operation of Reading-Side Radiation Imaging Apparatus 102)

In the reading-side radiation imaging apparatus 102 (to be also referred to as a reading apparatus hereinafter), the control unit 202e connects to the data storage unit 203 of the output-side radiation imaging apparatus 101 via the network 103 and obtains a protocol information group which is stored. The control unit 202e registers, in the protocol storage unit 202c, the obtained protocol information group as each imaging protocol of the reading-side radiation imaging apparatus 102 by associating the obtained protocol information group with the characteristic information of each imaging protocol registered in the protocol storage unit 202c.

In the reading-side radiation imaging apparatus 102, the display unit 202a can display, under the display control by the control unit 202b, a GUI for filtering or associating the characteristic information of each imaging protocol included in a synchronization protocol information group and the characteristic information of a corresponding imaging protocol registered in the reading-side radiation imaging apparatus 102. The display unit 202a, the control unit 202b, and the operation unit 202f function as an association unit that associates the characteristic information of another imaging protocol of another radiation imaging apparatus and the characteristic information of an imaging protocol stored in the protocol storage unit 202c. The control unit 202e controls the registration of the characteristic information of the other imaging protocol in the protocol storage unit 202c based on the association. More specifically, the association unit (the control unit 202b, the display unit 202a, and the operation unit 202f) associates the first characteristic information of the other imaging protocol of the other radiation imaging apparatus and the first characteristic information of a stored imaging protocol, and the control unit 202e controls the registration of the second characteristic information of the other imaging protocol in the protocol storage unit 202c based on the association.

### (Imaging Protocols and Protocol Information Group)

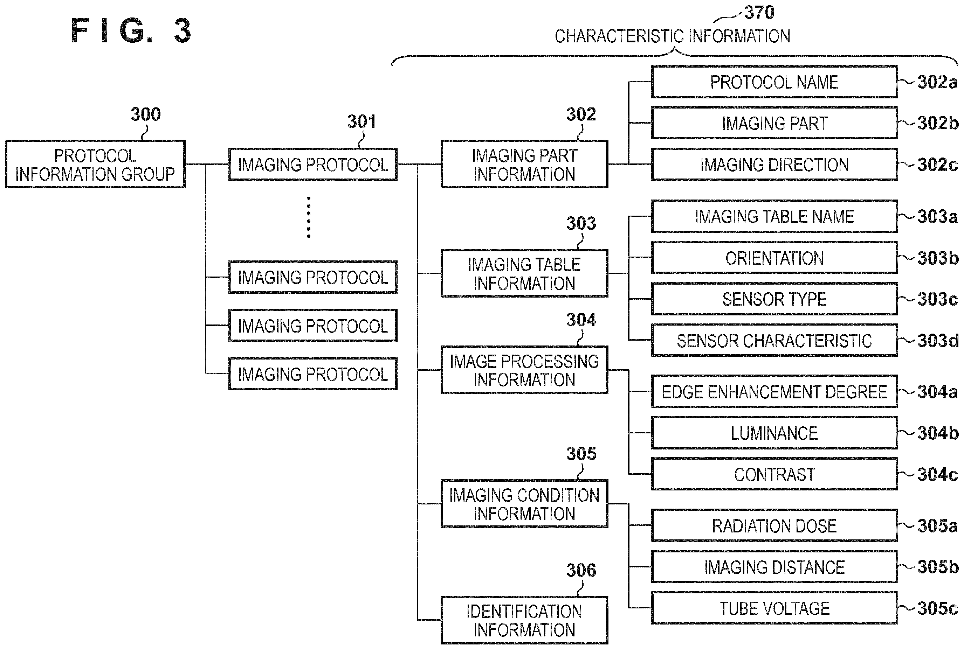

Fig. 3 is a block diagram showing an example of imaging protocols and a protocol information group. A protocol information group 300 includes at least one or more imaging protocols 301. Each imaging protocol 301 includes characteristic information 370 related to an imaging part, an imaging table, image processing, imaging conditions, and the like necessary for performing imaging on a subject. The characteristic information 370 includes at least one piece of imaging part information 302 of a subject who is to undergo the radiation imaging operation, imaging table information 303 which includes information of a detector to be used for the radiation imaging, image processing information 304 to be applied to an image obtained by the radiation imaging, imaging condition information 305 for controlling the radiation generating apparatus or the detector, and identification information 306 for identifying an imaging protocol from an external device.

The imaging part information 302 includes, for example, information such as a protocol name 302a for identifying and discriminating an imaging protocol, an imaging part 302b of the subject, and an imaging direction 302c (subject direction) of the subject.

The imaging table information 303 includes, information such as an imaging table name 303a, orientation 303b of the subject, sensor type 303c included in the imaging table, sensor characteristics 303d such as calibration information, and the like.

The image processing information 304 includes information related to image processing parameters such as an edge enhancement degree 304a, luminance 304b, contrast 304c, and the like to be applied to an image obtained by radiation imaging.

The imaging condition information 305 includes information for controlling the radiation generating apparatus and the detector (sensor). For example, the imaging condition information 305 includes information such as a radiation dose 305a, an imaging distance 305b, and a tube voltage 305c, and the like for controlling the imaging execution unit 201.

The identification information 306 includes information (for example, information such as a code value) for identifying the imaging protocol from the external device 104 such as an RIS.

When the imaging protocol 301 is to be shared between a plurality of radiation imaging apparatuses, there is information that needs to be excluded from the characteristic information 370 in order to perform imaging under different conditions for each radiation imaging apparatus. For example, since the imaging table 201b, which is shown in Fig. 2, differs for each radiation imaging apparatus, there may be a case in which the imaging table information 303 already held in the reading-side radiation imaging apparatus 102 is to be held without updating the imaging table information 303 based on the imaging table information obtained from the output-side radiation imaging apparatus 101. In this case, the imaging table information 303 can be excluded from the update target by using processing (to be described later), and the characteristic information 370 of the imaging protocol 301 from which the imaging table information 303 has been excluded can be shared between the output-side radiation imaging apparatus 101 and the reading-side radiation imaging apparatus 102.

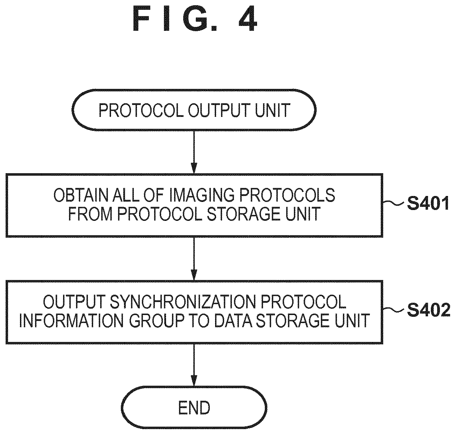

Fig. 4 is a flowchart for explaining the processing procedure in which the protocol output unit 202d of the output-side radiation imaging apparatus 101 stores a protocol information group in the data storage unit 203.

In step S401, the protocol output unit 202d obtains the set and registered protocol information group 300 (all of the imaging protocols) from the protocol storage unit 202c. In step S402, the protocol output unit 202d stores the obtained protocol information group 300 as the synchronization protocol information group 300 in the data storage unit 203. The synchronization protocol information group 300 represents the protocol information group 300 that is to be shared between the output-side radiation imaging apparatus 101 and the reading-side radiation imaging apparatus 102.

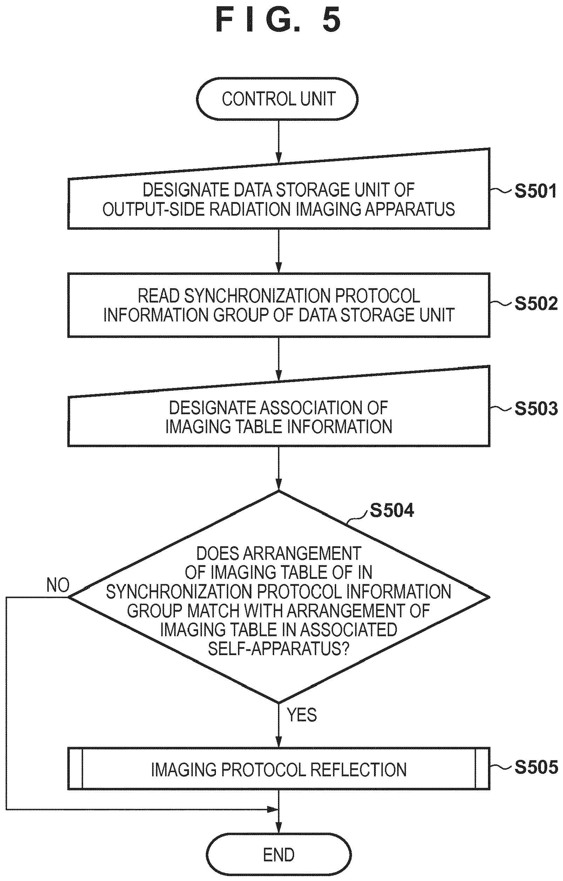

Fig. 5 is a flowchart for explaining the processing procedure in which the control unit 202e of the reading-side radiation imaging apparatus 102 stores the protocol information group 300 in the protocol storage unit 202c.

First, in step S501, a user designates the data storage unit 203 of the output-side radiation imaging apparatus 101 via the operation unit 202f. This can be implemented by designating the absolute path, the network address, or the apparatus name of the output-side radiation imaging apparatus 101.

Next, in step S502, the control unit 202e obtains the imaging protocol stored in the data storage unit 203 of another radiation imaging apparatus as the other imaging protocol of the other radiation imaging apparatus. More specifically, the control unit 202e communicates with the data storage unit 203 of the designated output-side radiation imaging apparatus 101, obtains the synchronization protocol information group stored in the data storage unit 203 in step S402, and reads the synchronization protocol information group.

In step S503, the association unit (control unit 202b, the display unit 202a, and the operation unit 202f) performs association based on the obtained imaging protocol. More specifically, the user associates, via the operation unit 202f, the characteristic information 370 of the imaging protocol 301 and the synchronization protocol information group 300 read from the output-side radiation imaging apparatus 101. The imaging table information 303 is used as an example to describe the association of the characteristic information 370 hereinafter. However, the present invention is not limited to this example, and it is possible to associate and register other pieces of information included in the characteristic information 370. This step designates the association between the characteristic information 370 (for example, the imaging table information 303) of each output-side imaging protocol 301 and the characteristic information 370 (for example, the imaging table information 303) of each imaging protocol registered in the protocol storage unit 202c of the reading-side radiation imaging apparatus 102.

In step S504, the control unit 202e compares, based on the association by the association unit (the control unit 202b, the display unit 202a, and the operation unit 202f), the characteristic information of the imaging protocol and the characteristic information of the other imaging protocol, and controls the registration (reading) of the imaging table information based on the comparison result. If the arrangement of the imaging table included in the synchronization protocol information group does not match with the arrangement of the imaging table associated in step S503 (NO in step S504), the control unit 202e stops the processing. Otherwise (YES in step S504), the control unit continues the processing.

The arrangement of the imaging table can be determined by the type and the number of imaging tables. As the imaging table type, it is possible to handle imaging tables with sensors to be used for imaging that have at least matching imaging sizes as imaging tables of the same type. If an imaging protocol is shared between radiation imaging apparatuses with different numbers and types of imaging tables, the association designation performed in step S503 becomes an incomplete association designation. Such a case can result in an imaging protocol that cannot be shared or an imaging protocol that cannot be used for imaging even if it is shared. That is, this embodiment assumes a case in which the operation of the output-side radiation imaging apparatus 101 and the reading-side radiation imaging apparatus 102 match.

In the determination of step S504, if the arrangements of the imaging tables match (YES in step S504), the process advances to step S505, and imaging protocol reflection is performed.

In step S505, if the characteristic information of the imaging protocol and the characteristic information of the other imaging protocol match, the control unit 202e replaces the characteristic information of the stored imaging protocol with the characteristic information of the other imaging protocol and stores and registers the characteristic information of the other imaging protocol in the protocol storage unit 202c. A more specific description of the imaging protocol reflection processing will be given later.

### (Imaging Protocol Reflection Processing)

The control unit 202e deletes all of the imaging protocols stored in the protocol storage unit 202c and stores, in the protocol storage unit 202c, each imaging protocol that has been obtained by replacing, based on the association, the characteristic information of the imaging protocol stored in the protocol storage unit 202c with the characteristic information of the other imaging protocol.

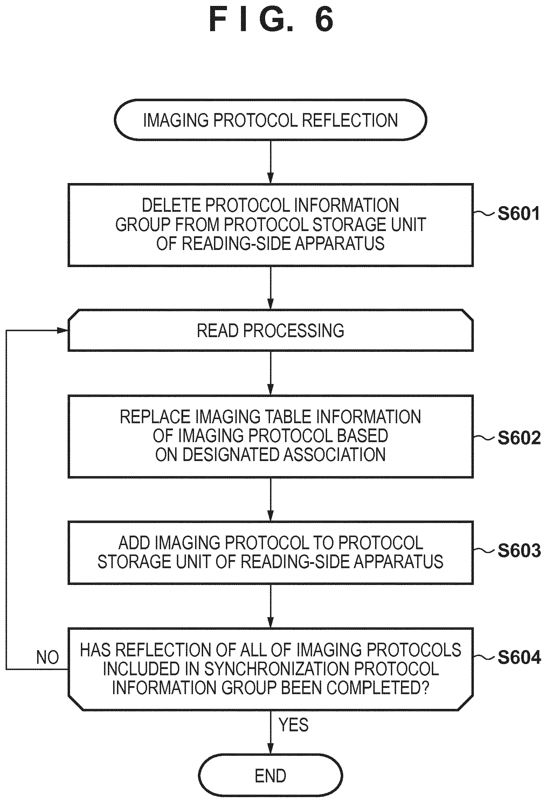

Fig. 6 is a flowchart for explaining a more specific processing procedure of the imaging protocol reflection processing performed in step S505 of Fig. 5.

In step S601, when imaging protocol reflection is started, the control unit 202e deletes each imaging protocol registered in the protocol storage unit 202c (step S601).

In step S602, based on the synchronization protocol information group 300 obtained from the output-side radiation imaging apparatus 101, the control unit 202e replaces the imaging table information 303 of the processing target imaging protocol based on the association designated in step S503 of Fig. 5. As a result, the processing target imaging protocol can be used for imaging by the reading-side radiation imaging apparatus 102.

In step S603, the control unit 202e adds the imaging protocol of the imaging table information 303 replaced in step S602 to the protocol storage unit 202c of the reading-side radiation imaging apparatus 102.

Subsequently, in step S604, the control unit 202e repetitively performs imaging protocol reflection processing of steps S602 and S603 for all of the imaging protocols 301 included in the synchronization protocol information group 300. This processing ends when the reflection processing of all of the imaging protocols included in the synchronization protocol information group 300 has been completed.

### (Association Designation GUI)

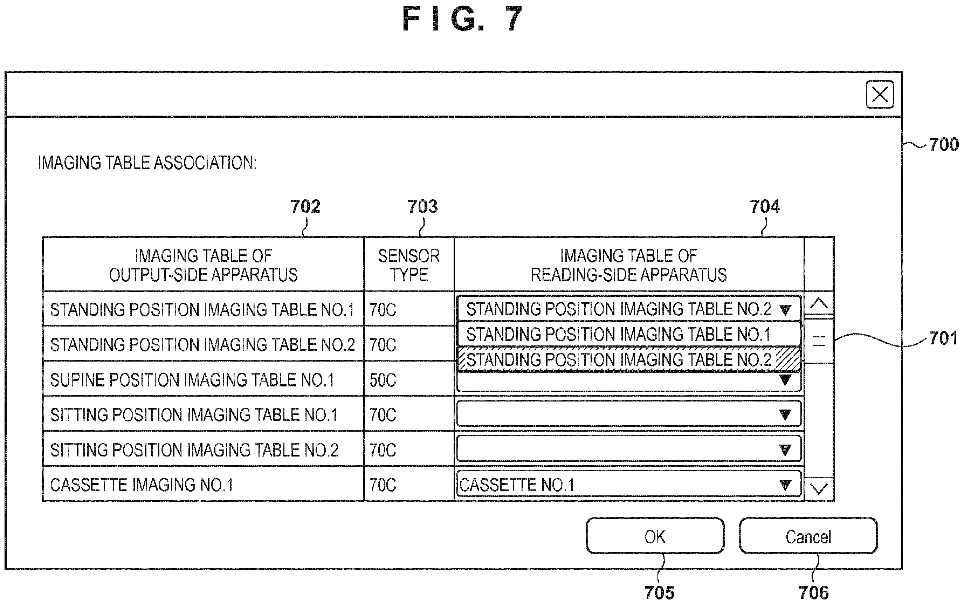

The association unit (the control unit 202b, the display unit 202a, and the operation unit 202f) performs display control to cause the control unit 202b to display an association screen for making the association. Fig. 7 is a view showing an example of a GUI (an imaging table association screen) for designating the association between pieces of imaging table information 303 in step S503 of Fig. 5. An association screen 700 (GUI) for designating the association of Fig. 7 is display controlled by the control unit 202b.

When the user instructs, via the operation unit 202f, the reading of each imaging protocol, the association screen 700 (GUI) for the characteristic information 370 (for example, the imaging table information 303) is displayed on the display unit 202a of the reading-side radiation imaging apparatus 102. An imaging table association list 701 for associating the pieces of imaging table information is displayed on the imaging table association screen 700. The imaging table association list 701 includes a list 702 of the imaging table information 303 of the output-side radiation imaging apparatus 101 included in the synchronization protocol information group 300 that was read in step S502 of Fig. 5, a sensor type display portion 703 which displays the sensor types of sensors included in the respective imaging tables displayed in the list 702 of the imaging table information 303, and an association portion 704 for setting the imaging table information of the reading-side radiation imaging apparatus 102 that is to be associated with the corresponding piece of imaging table information 303 displayed on the list 702.

In the association portion 704, among the pieces of imaging table information 303 included in the imaging protocols 301 set and registered in the protocol storage unit 202c of the reading-side radiation imaging apparatus 102, each piece of imaging table information that has a sensor type matching the sensor type displayed on the sensor type display portion 703 is displayed in a combo box or the like under selectable display control.

The association portion 704 associates the imaging table information 303 of the output-side radiation imaging apparatus displayed in the list 702 with the imaging table information of the reading-side radiation imaging apparatus 102 based on the input from the operation unit 202f, and the association designation of the imaging table information 303 of the reading-side radiation imaging apparatus 102 is completed when the user presses an association complete button 705 (OK). When a reading cancellation instruction button 706 (Cancel) is pressed, the imaging protocol reflection processing is not started, and the imaging table association screen 700 is discarded.

As described above, when an imaging protocol of the output-side radiation imaging apparatus 101 is to be reflected on the reading-side radiation imaging apparatus 102, which is another radiation imaging apparatus, it is possible to register the imaging protocol as an imaging protocol to be used for imaging in the reading-side radiation imaging apparatus 102 by, for example, replacing the imaging table information of the reading-side radiation imaging apparatus 102 with the imaging table information 303 based on the imaging protocol of the output-side radiation imaging apparatus 101.

According to this embodiment, it is possible to set a suitable imaging protocol for each radiation imaging apparatuses by associating the characteristic information of the sharing target imaging protocol with the corresponding characteristic information that can be applied in the reading-side radiation imaging apparatus.

### [Second Embodiment]

This embodiment will describe an example in which an output-side radiation imaging apparatus 101 outputs a protocol information group 300 and stores the protocol information group in a data storage unit 203 of a reading-side radiation imaging apparatus 102. Note that, to avoid redundancy, a description of parts in common with the arrangement of the first embodiment will be omitted.

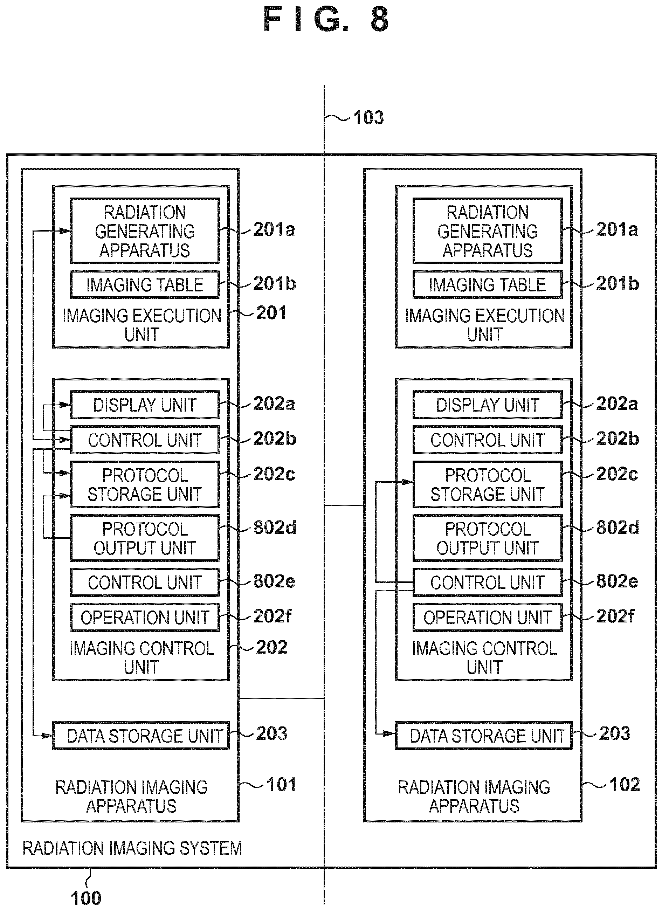

Fig. 8 is a block diagram showing the system arrangement of a radiation imaging system 100 according to the second embodiment to which the present invention is applied. A protocol output unit 802d outputs each imaging protocol stored in a protocol storage unit 202c. The protocol output unit 802d of the output-side radiation imaging apparatus 101 obtains all of the imaging protocols stored in the protocol storage unit 202c, and stores all of the obtained imaging protocols in the data storage unit 203 of another radiation imaging apparatus.

The protocol output unit 802d of the output-side radiation imaging apparatus 101 obtains the protocol information group 300 (all of the imaging protocols) that has been set and registered by the protocol storage unit 202c. The protocol output unit 802d stores the obtained protocol information group 300 in the data storage unit 203 of the reading-side radiation imaging apparatus 102 via the network 103.

In the reading-side radiation imaging apparatus 102, in order to share each imaging protocol 301 included in the protocol information group 300, a control unit 802e obtains the protocol information group stored in the data storage unit 203 of the reading-side radiation imaging apparatus 102. Subsequently, the control unit 802e sets and registers the obtained protocol information group in the protocol storage unit 202c of the reading-side radiation imaging apparatus 102.

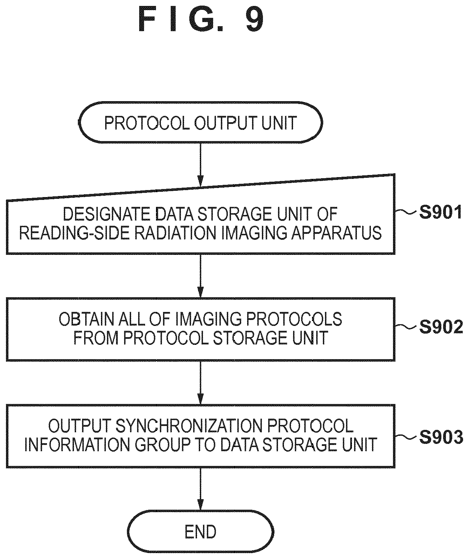

Fig. 9 is a flowchart for explaining the processing procedure in which the protocol output unit 802d of the output-side radiation imaging apparatus 101 stores the protocol information group 300 in the data storage unit 203 of the reading-side radiation imaging apparatus 102.

In step S901, the protocol output unit 802d of the output-side radiation imaging apparatus 101 accepts the user designation of the data storage unit 203 of reading-side radiation imaging apparatus 102. This is implemented by a user designating the absolute path, the network address, or the apparatus name of the radiation imaging apparatus.

In step S902, the protocol output unit 802d obtains, from the protocol storage unit 202c, the protocol information group (all of the imaging protocols) set in the output-side radiation imaging apparatus 101.

In step S903, the protocol output unit 802d stores the obtained protocol information group 300 as the synchronization protocol information group in the data storage unit 203 of the reading-side radiation imaging apparatus 102. The synchronization protocol information group represents the protocol information group that is to be shared between the output-side radiation imaging apparatus 101 and the reading-side radiation imaging apparatus 102.

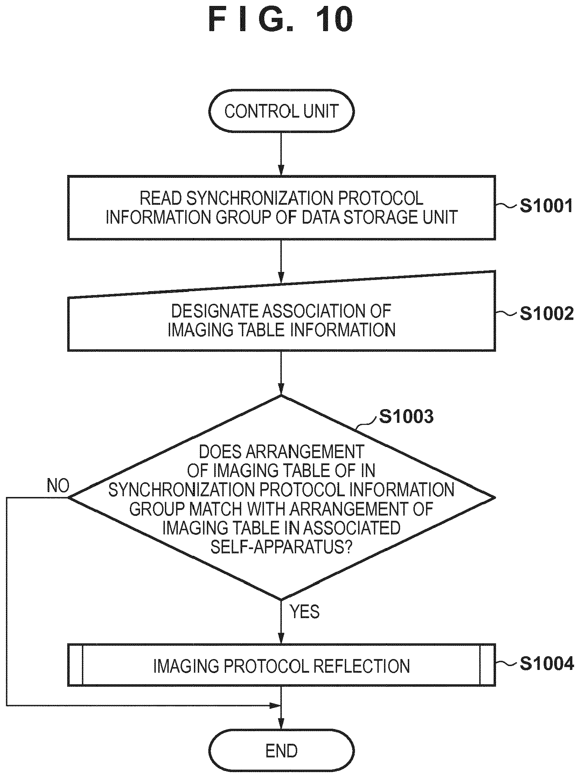

Fig. 10 is a flowchart for explaining the processing procedure in which the control unit 802e of the reading-side radiation imaging apparatus 102 stores the protocol information group in the protocol storage unit 202c.

In step S1001, the control unit 802e reads, from the data storage unit 203 of the reading-side radiation imaging apparatus 102, the synchronization protocol information group which was output from the output-side radiation imaging apparatus 101 in step S903.

In step S1002, the user designates, via the operation unit 202f, the association between imaging table information 303 included in the synchronization protocol information group which was read from the output-side radiation imaging apparatus 101 and the imaging table information 303 included in the imaging protocol registered in the protocol storage unit 202c.

In step S1003, if the arrangement of the imaging table included in the synchronization protocol information group and the arrangement of the imaging table associated in step S1002 do not match (NO in step S1003), the control unit 802e stops the processing. If the arrangements of the imaging tables match (YES in step S1003), the process advances to step S1004.

In step S1004, imaging protocol reflection processing is performed. The imaging protocol reflection processing is the same processing as that described in Fig. 6.

According to this embodiment, it is possible to perform imaging protocol sharing regardless of whether the output-side radiation imaging apparatus can be accessed from the reading-side radiation imaging apparatus by causing the output-side radiation imaging apparatus 101 to output a synchronization protocol information group to the data storage unit of the reading-side radiation imaging apparatus.

### [Third Embodiment]

This embodiment will describe an arrangement in which a synchronization protocol information group is stored in a storage area that is not a part of a radiation imaging system 100. Note that, to avoid redundancy, a description of parts in common with the arrangements of the first and second embodiments will be omitted.

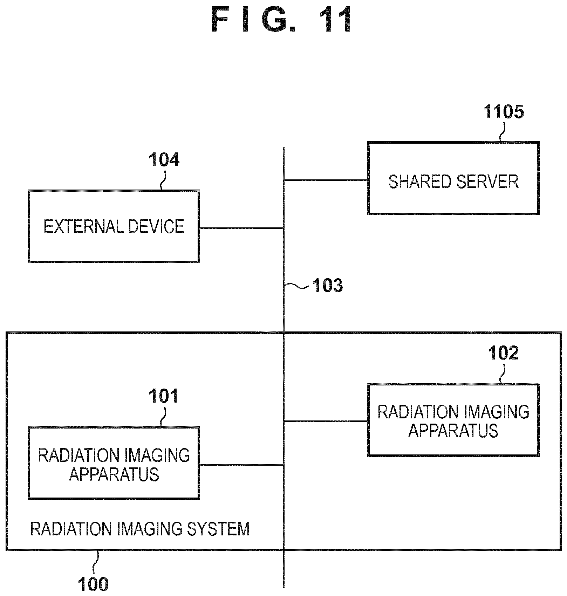

Fig. 11 is a block diagram showing the arrangement of a radiation imaging system 100 according to the third embodiment to which the present invention is applied. A protocol output unit 1202d obtains all of the imaging protocols stored in a protocol storage unit 202c, and stores all of the obtained imaging protocols in a shared server which is connected via a network 103. The radiation imaging system 100 includes at least one output-side radiation imaging apparatus 101 and at least one reading-side radiation imaging apparatus 102, and the plurality of radiation imaging apparatuses 101 and 102 are connected to each other via the network 103. The radiation imaging system 100 is also connected, via the network 103, to an external device 104 such as an RIS and a shared server 1105 for data storage.

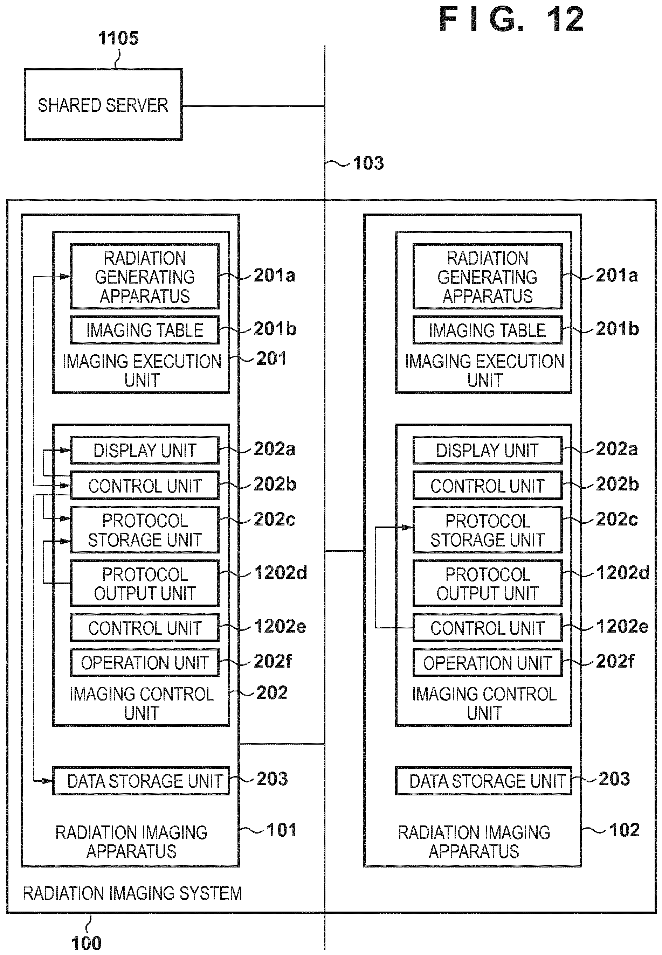

Fig. 12 is a block diagram showing the functional arrangement of the radiation imaging system 100. The protocol output unit 1202d of the output-side radiation imaging apparatus 101 obtains a protocol information group 300 which has been set and registered by the protocol storage unit 202c, and stores the obtained protocol information group 300 in the shared server 1105 via the network 103. In the reading-side radiation imaging apparatus 102, in order to share each imaging protocol 301 included in the protocol information group 300, a control unit 1202e of the reading-side radiation imaging apparatus 102 accesses the shared server 1105, obtains the protocol information group 300 stored in the shared server 1105, and stores the obtained protocol information group 300 in the protocol storage unit 202c of the reading-side radiation imaging apparatus 102.

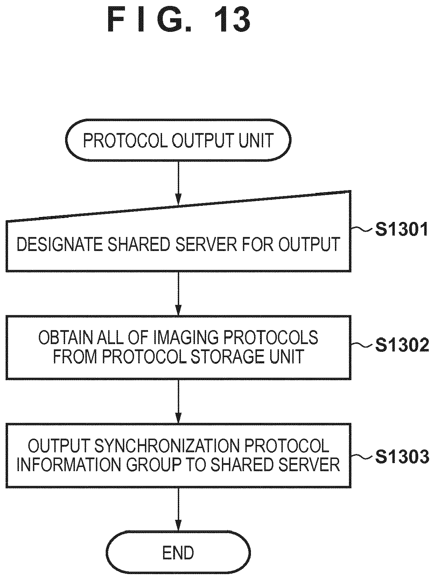

Fig. 13 is a flowchart for explaining the processing procedure in which the protocol output unit 1202d of the output-side radiation imaging apparatus 101 stores the protocol information group in the shared server 1105.

In step S1301, the protocol output unit 1202d designates the shared server 1105 under the operation by the user. Next, in step S1302, the protocol output unit 1202d obtains the protocol information group 300 (all of the imaging protocols) set in the output-side radiation imaging apparatus 101 from the protocol storage unit 202c. In step S1303, the protocol output unit 1202d stores the obtained protocol information group 300 (all of the imaging protocols) as the synchronization protocol information group 300 in the shared server 1105.

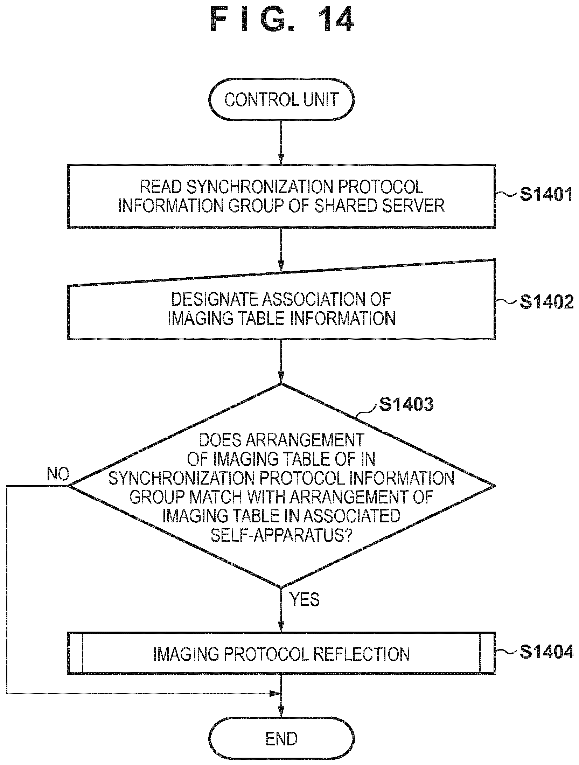

The control unit 1202e obtains each imaging protocol stored in the shared server 1105 as another imaging protocol for another radiation imaging apparatus, and an association unit (a control unit 202b, a display unit 202a, and an operation unit 202f) performs association based on the obtained imaging protocols. Fig. 14 is a flowchart for explaining the processing procedure in which the control unit 1202e of the reading-side radiation imaging apparatus 102 stores the protocol information group 300 in the protocol storage unit 202c of the reading-side radiation imaging apparatus 102.

In step S1401, the control unit 1202e of the reading-side radiation imaging apparatus 102 reads, from the shared server 1105, the synchronization protocol information group 300 which was output from the output-side radiation imaging apparatus 101 in step S1303 of Fig. 13.

In step S1402, the user designates, via the operation unit 202f, the association between imaging table information 303 of the imaging protocol 301 included in the read synchronization protocol information group 300 and the imaging table information 303 included in the imaging protocol 301 which is registered in the protocol storage unit 202c of the reading-side radiation imaging apparatus 102. At this time, if the arrangement of the imaging table in the imaging protocol 301 included the synchronization protocol information group 300 does not match with the arrangement of the imaging table associated in step S1402 (NO in step S1402), the control unit stops the processing. If the arrangements of the imaging tables match (YES in step S1403), the process advances to step S1404.

In step S1404, imaging protocol reflection processing is performed. The imaging protocol reflection processing is the same processing as that described in Fig. 6.

According to this embodiment, by separating the output location of the protocol information group from the radiation imaging system, imaging protocol sharing can be performed without influencing the operations of the output-side radiation imaging apparatus and the reading-side radiation imaging apparatus.

### [Fourth Embodiment]

This embodiment will describe an example of the arrangement in which a registration target is selected (filtered) on an imaging protocol 301 basis at the reading of a synchronization protocol information group 300. Note that, to avoid redundancy, a description of parts in common with the arrangements of the first to third embodiments will be omitted. The output location of the synchronization protocol information group in this embodiment may be the reading-side radiation imaging apparatus 102 described in the above embodiments or the shared server 1105 for data storage described in the third embodiment.

In this embodiment, a display unit 202a, a control unit 202b, and an operation unit 202f function as a registration selection unit for selecting information to be registered in a protocol storage unit 202c based on a setting. Among other imaging protocols which have been associated by an association unit, a registration selection unit (the display unit 202a, the control unit 202b, and the operation unit 202f) stores each imaging protocol selected by the registration selection unit in the protocol storage unit 202c.

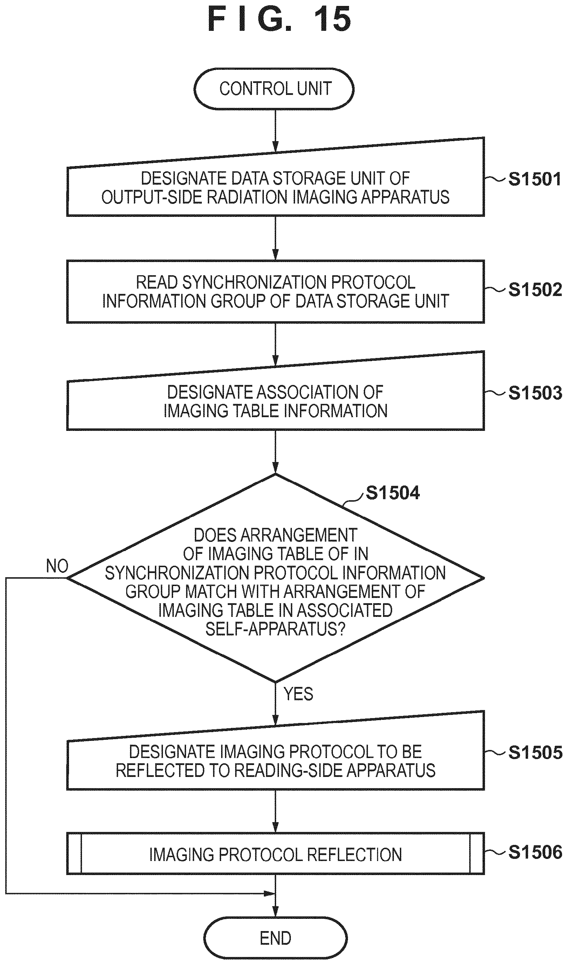

Fig. 15 is a flowchart for explaining the processing procedure in which a control unit 202e of a reading-side radiation imaging apparatus 102 selects a registration target on an imaging protocol basis, and stores the selected imaging protocol of the protocol information group 300 in the protocol storage unit 202c.

In step S1501, a user designates a data storage unit 203 of an output-side radiation imaging apparatus 101 via the operation unit 202f of the reading-side radiation imaging apparatus 102. This is implemented by designating the absolute path, the network address, or the apparatus name of the radiation imaging apparatus.

In step S1502, the control unit 202e communicates with the designated data storage unit 203 and obtains and reads the synchronization protocol information group 300 stored in the data storage unit 203 of the output-side radiation imaging apparatus 101.

In step S1503, the user designates, via the operation unit 202f, the association between imaging table information 303 of the imaging protocol 301 included in the synchronization protocol information group 300 which was read from the output-side radiation imaging apparatus 101 and the imaging table information 303 included in the imaging protocol 301 which is registered in the protocol storage unit 202c of the reading-side radiation imaging apparatus 102.

At this time, if the arrangement of the imaging table in the imaging protocol 301 included in the synchronization protocol information group 300 and the arrangement of the imaging table associated in step S1503 do not match (NO in step S1504), the processing is stopped. If the arrangements of the imaging tables match (YES in step S1504), the process advances to step S1505.

In step S1505, the user selects, via the operation unit 202f, each imaging protocol to be stored in the reading-side radiation imaging apparatus 102 from the imaging protocols included in the synchronization protocol information group 300.

In step S1506, imaging protocol reflection processing is performed. The imaging protocol reflection processing will be described later.

### (Imaging Protocol Reflection Processing)

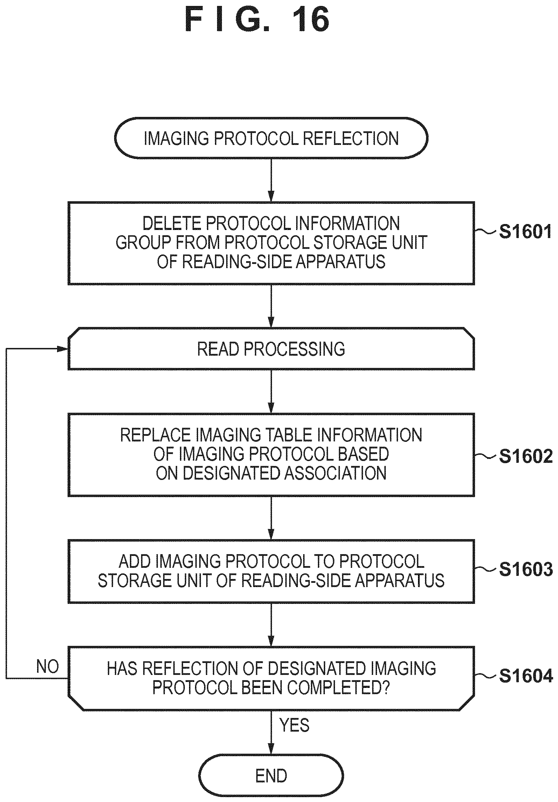

Fig. 16 is a flowchart for explaining a more specific processing procedure of the imaging protocol reflection processing in step S1506 of Fig. 15.

In step S1601, when the imaging protocol reflection processing is started, the control unit 202e deletes the imaging protocols registered in the protocol storage unit 202c of the reading-side radiation imaging apparatus 102.

In step S1602, the control unit 202e replaces, based on the association designated in step S1503 of Fig. 15, each processing target imaging protocol with the imaging protocol selected in step S1505. As a result, each processing target imaging protocol can be used for imaging in the reading-side radiation imaging apparatus 102.

In step S1603, the control unit 202e stores, in the protocol storage unit 202c of the reading-side radiation imaging apparatus 102, each imaging protocol that was replaced in the step S1602.

In step S1604, the control unit 202e repetitively performs the processes of steps S1602 and S1603 on each imaging protocol that was selected in step S1505.

### (Imaging Protocol Selection GUI)

The registration selection unit (the display unit 202a, the control unit 202b, and the operation unit 202f) performs display control to cause the display unit 202a to display an imaging protocol selection screen (GUI) for setting information that is to be registered in the protocol storage unit 202c.

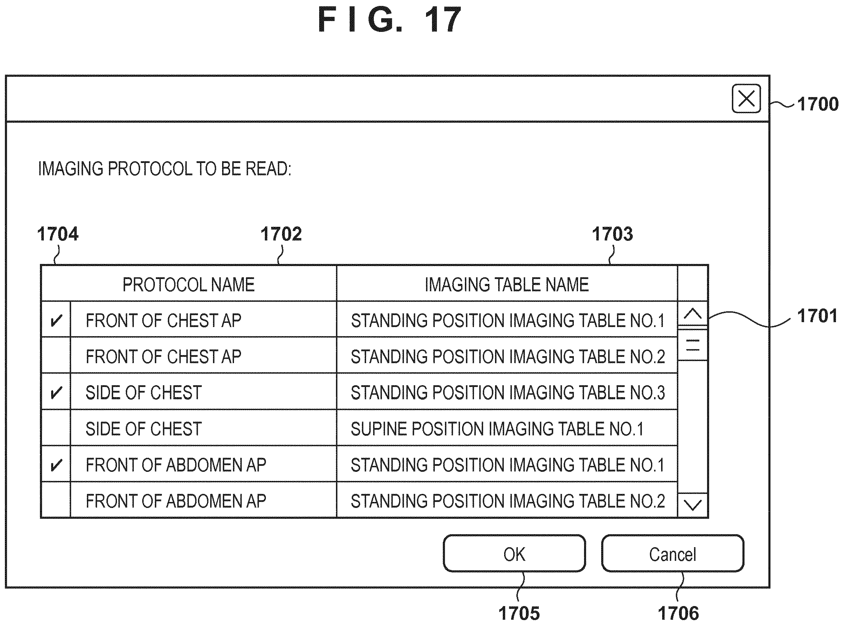

Fig. 17 is a view showing an example of an imaging protocol selection screen 1700 (GUI) for selecting each imaging protocol in step S1505 of Fig. 15. The imaging protocol selection screen 1700 (GUI) is displayed under the control of the control unit 202b.

For example, when an association complete button 705 (OK) is pressed on an imaging table association screen 700 shown in Fig. 7, the imaging protocol selection screen 1700 is displayed under the control of the control unit 202b. An imaging protocol selection list 1701 for selecting each registration target imaging protocol is displayed on the imaging protocol selection screen 1700.

The imaging protocol selection list 1701 includes a list display portion 1702 that displays a list of imaging protocols included in the synchronization protocol information group 300.

The imaging protocol selection list 1701 includes an imaging table information display portion 1703 that displays each piece of imaging table information that has been obtained by replacing the imaging table information included in the imaging protocol displayed in the list display portion 1702 based on the association performed on the association screen 700.

The imaging protocol selection list 1701 also includes a shared imaging protocol selection portion 1704 for setting whether to select each imaging protocol which is shown in the list display portion 1702 and the imaging table information display portion 1703 as a registration target.

The user selects, via the operation unit 202f, the imaging protocol to be set as a registration target in the shared imaging protocol selection portion 1704, and the imaging protocol reflection processing described in Fig. 16 is executed when the user presses a selection complete button 1705 (OK). When the user presses a selection cancellation instruction button 1706 (Cancel), the imaging protocol reflection processing is not executed, and the imaging protocol selection screen 1700 is discarded.

According to this embodiment, by selecting each imaging protocol to be reflected on the reading-side radiation imaging apparatus 102 (self-apparatus) at the time of the registration (reading), it becomes possible to share only the imaging protocol necessary for the self-apparatus with the output-side radiation imaging apparatus 101.

### [Fifth Embodiment]

This embodiment will describe an arrangement in which an output target is selected (filtered) on an imaging protocol basis at the time of the output of a synchronization protocol information group in the output-side radiation imaging apparatus 101. Note that, to avoid redundancy, a description of parts in common with the above-described first to fourth embodiments will be omitted. Note that the output location of the synchronization protocol information group in this embodiment may be the reading-side radiation imaging apparatus 102 described in the above embodiments or the shared server 1105 for data storage described in the third embodiment.

In this embodiment, a display unit 202a, a control unit 202b, and an operation unit 202f function as an output selection unit for selecting, among all of the imaging protocols stored in a protocol storage unit 202c, each imaging protocol which is to be output outside. Among all of the imaging protocols that have been obtained from the protocol storage unit 202c, the output selection unit (the display unit 202a, the control unit 202b, and the operation unit 202f) selects each imaging protocol to be output outside based on a setting and stores each selected imaging protocol in a data storage unit 203.

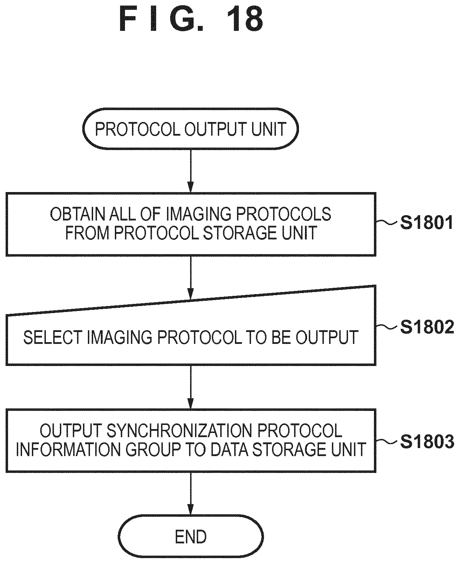

Fig. 18 is a flowchart for explaining processing procedure in which the output-side radiation imaging apparatus 101 selects, via a protocol output unit 202d, each imaging protocol to be output on an imaging protocol basis and stores a protocol information group which includes the selected imaging protocol in the data storage unit 203.

In step S1801, the protocol output unit 202d obtains a protocol information group 300 that has been set and registered from the protocol storage unit 202c. Next, in step S1802, a user selects, by using an imaging protocol selection screen (GUI) for selecting at least one imaging protocol from the protocol information group 300, one imaging protocol 301 or a plurality of imaging protocols 301 which are to be output as synchronization protocol information or a synchronization protocol group from the protocol information group 300 obtained in step S1801.

In step S1803, the protocol output unit 202d outputs, to the data storage unit 203, the plurality of imaging protocols 301 or the one imaging protocol 301 that has been selected on the imaging protocol selection screen (GUI) as the synchronization protocol information group or the synchronization protocol information. The synchronization information that has been selected (filtered) on an imaging protocol basis via the GUI is stored in the data storage unit 203.

The output selection unit (the display unit 202a, the control unit 202b, and the operation unit 202f) performs display control to cause the display unit 202a to display an imaging protocol selection screen (GUI) for setting each imaging protocol to be output outside.

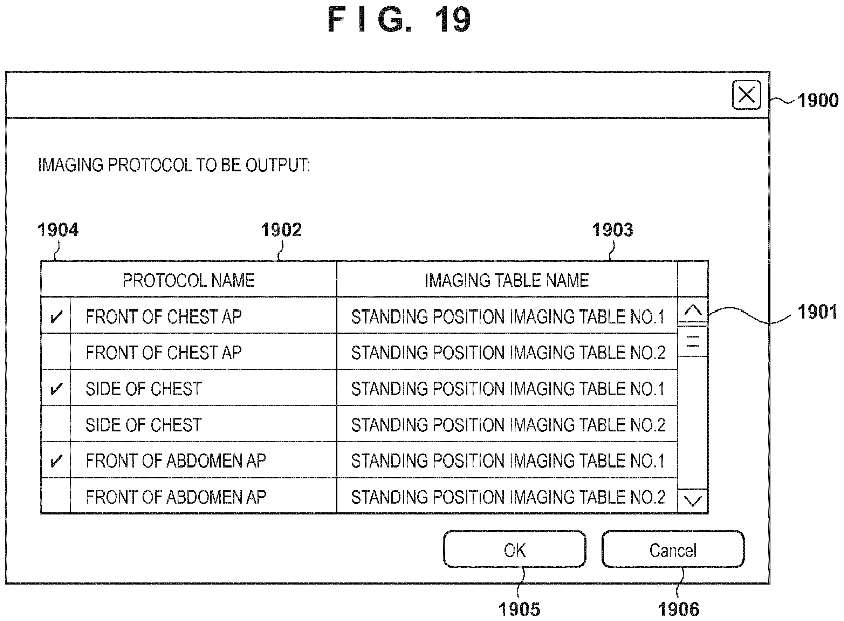

Fig. 19 is a view showing an example of an imaging protocol selection screen 1900 (GUI) for selecting each imaging protocol to be used in step S1802. When the protocol output unit 202d obtains a protocol information group registered in the protocol storage unit 202c, the imaging protocol selection screen 1900 is displayed under the control of the control unit 202b.

An imaging protocol selection list 1901 for selecting each imaging protocol to be output is displayed on the imaging protocol selection screen 1900.

The imaging protocol selection list 1901 includes an imaging protocol display portion 1902 that shows a list of imaging protocols 301 included in the protocol information group 300 obtained in step S1801.

The imaging protocol selection list 1901 also includes an imaging table information display portion 1903 that displays imaging table information which is included in each imaging protocol displayed in the imaging protocol display portion 1902.

The imaging protocol selection list 1901 includes an imaging protocol selection portion 1904 for setting whether to select each imaging protocol which is shown in the imaging protocol display portion 1902 and the imaging table information display portion 1903 as an output target.

When the user selects, on the imaging protocol selection portion 1904 via the operation unit 202f, each imaging protocol to be an output target, and presses a imaging protocol selection complete button 1905 (OK), processing to output the plurality of imaging protocols 301 (protocol information group) or the one imaging protocol 301 (protocol information) selected on the imaging protocol selection screen 1900 as synchronization information to the data storage unit 203 is executed. When a selection cancellation instruction button 1906 (Cancel) is pressed, processing to output the imaging protocol 301 and the like to the data storage unit 203 is not executed, and the imaging protocol selection screen 1900 is discarded.

According to this embodiment, by selecting each imaging protocol to be a sharing target at the time of output, it is possible to share only the imaging protocols which are necessary as sharing targets by excluding, in advance, each imaging protocol which is unnecessary in another apparatus (a reading-side radiation imaging apparatus 102) on the side of the output-side radiation imaging apparatus 101 even in case in which apparatus-specific imaging protocols are present in the output-side radiation imaging apparatus 101. As a result, it is possible to reduce, on the side of the reading-side radiation imaging apparatus 102, a user's load when he/she has to select the necessary imaging protocols when imaging protocols are to be shared.

The arrangement of this embodiment may be combined with the arrangement of the reading-side radiation imaging apparatus 102 according to the fourth embodiment described above. That is, at the time at which the synchronization protocol information group is to be output from the output-side radiation imaging apparatus 101, the output target information may be filtered on an imaging protocol basis, and the registration target information may be selected on an imaging protocol basis when the synchronization protocol information group is read by the reading-side radiation imaging apparatus 102.

By combing the arrangement of this embodiment with the arrangement of the fourth embodiment, in addition to the above-described effect of performing filtering in the output-side radiation imaging apparatus 101, it is possible to select the imaging protocols to be reflected to the reading-side radiation imaging apparatus 102 (self-apparatus) at the time of reading so that only imaging protocols which are necessary for the self-apparatus are shared with the output-side radiation imaging apparatus 101.

### [Sixth Embodiment]

This embodiment will describe an arrangement in which a reading-side radiation imaging apparatus 102 (self-apparatus) does not delete but holds each imaging protocol which has been uniquely registered in the self-apparatus, and newly additionally registers each imaging protocol other than the imaging protocols already registered in the self-apparatus. Note that, to avoid redundancy, a description of parts in common with the arrangements of the first to fifth embodiments will be omitted.

A control unit 202e holds each imaging protocol stored in a protocol storage unit 202c and stores, in the protocol storage unit 202c, each imaging protocol obtained by replacing the characteristic information of the imaging protocol stored in the protocol storage unit 202c with the characteristic information of another imaging protocol.

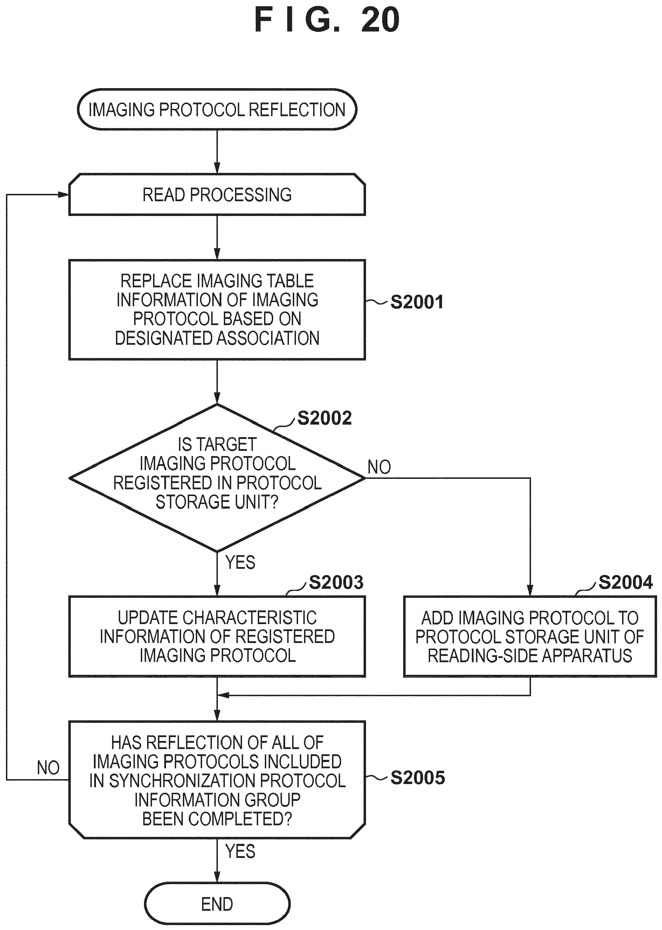

Fig. 20 is a flowchart for explaining a more specific processing procedure of the imaging protocol reflection processing described in step S505 of Fig. 5.

In step S2001, when imaging protocol reflection processing is started, the control unit 202e replaces, based on the association designated in step S503 of Fig. 5, the characteristic information (for example, imaging table information 303) of a processing target imaging protocol based on an imaging protocol obtained from an output-side radiation imaging apparatus 101. As a result, the processing target imaging protocol can be used for imaging by the reading-side radiation imaging apparatus 102.

If an imaging protocol that has been associated with the characteristic information by an association unit is stored in the protocol storage unit 202c (step S2002), the control unit 202e updates (step S2003), based on the association, the characteristic information of the imaging protocol registered in the protocol storage unit 202c.

More specifically, in step S2002, the control unit 202e determines whether an imaging protocol whose characteristic information (for example, the imaging table information 303) has been replaced is already registered in the protocol storage unit 202c of the reading-side radiation imaging apparatus 102. The control unit 202e determines whether the imaging protocol is already registered in the protocol storage unit 202c based on the characteristic information included in the imaging protocol. For example, this can be determined by determining whether an imaging protocol matching a protocol name 302a of the processing target imaging protocol and the imaging table information 303 of the characteristic information is present in the protocol storage unit 202c of the reading-side radiation imaging apparatus 102.

In the determination of step S2002, if an imaging protocol whose characteristic information (for example, the imaging table information 303) has been replaced is already registered in the protocol storage unit 202c of the reading-side radiation imaging apparatus 102 (YES in step S2002), the process advances to step S2003. Next, in step S2003, the control unit 202e updates the characteristic information of the already registered imaging protocol by using the characteristic information of the processing target imaging protocol.

On the other hand, in the determination of step S2002, if an imaging protocol whose characteristic information has been associated by the association unit is not stored in the protocol storage unit 202c, the control unit 202e adds the imaging protocol whose characteristic information has been associated in the protocol storage unit 202c. More specifically, if an imaging protocol whose characteristic information (for example, the imaging table information 303) has been replaced is not registered in the protocol storage unit 202c of the reading-side radiation imaging apparatus 102 (NO in step S2002), the process advances to step S2004. Next, in step S2004, the control unit 202e adds the processing target imaging protocol whose characteristic information has been replaced in the protocol storage unit 202c of the reading-side radiation imaging apparatus 102.

In step S2005, the control unit 202e repetitively performs the imaging protocol reflection processing of steps S2001 to S2004 on every imaging protocol included in the synchronization protocol information group. Subsequently, the processing ends when the reflection processing of all of the imaging protocols included in the synchronization protocol information group is completed.

According to this embodiment, it is possible to hold without deleting the imaging protocols uniquely added to and already registered in the reading-side radiation imaging apparatus 102 (self-apparatus), while newly adding and registering each imaging protocol other than those already registered in the self-apparatus. That is, by excluding, from the deletion target, and holding each imaging protocol that has been uniquely added and registered in the reading-side radiation imaging apparatus 102 (self-apparatus), it becomes possible to add and register (update), while holding each unique imaging protocol, the characteristic information of each imaging protocol that is shared between the output-side radiation imaging apparatus 101 and the reading-side radiation imaging apparatus 102 by using the characteristic information of the synchronization protocol information group, and thus the influence on the reading-side radiation imaging apparatus 102 can be reduced.

### [Seventh Embodiment]

This embodiment will describe an arrangement which filters the characteristic information to be added and registered (updated) in a reading-side radiation imaging apparatus 102 (self-apparatus) when each imaging protocol is to be read from an output-side radiation imaging apparatus 101 in the above-described sixth embodiment.

In this embodiment, a display unit 202a, a control unit 202b, and an operation unit 202f function as an update selection unit that selects (filters) the characteristic information of an imaging protocol to be updated. A control unit 202e updates the characteristic information based on the characteristic information selected by the update selection unit.

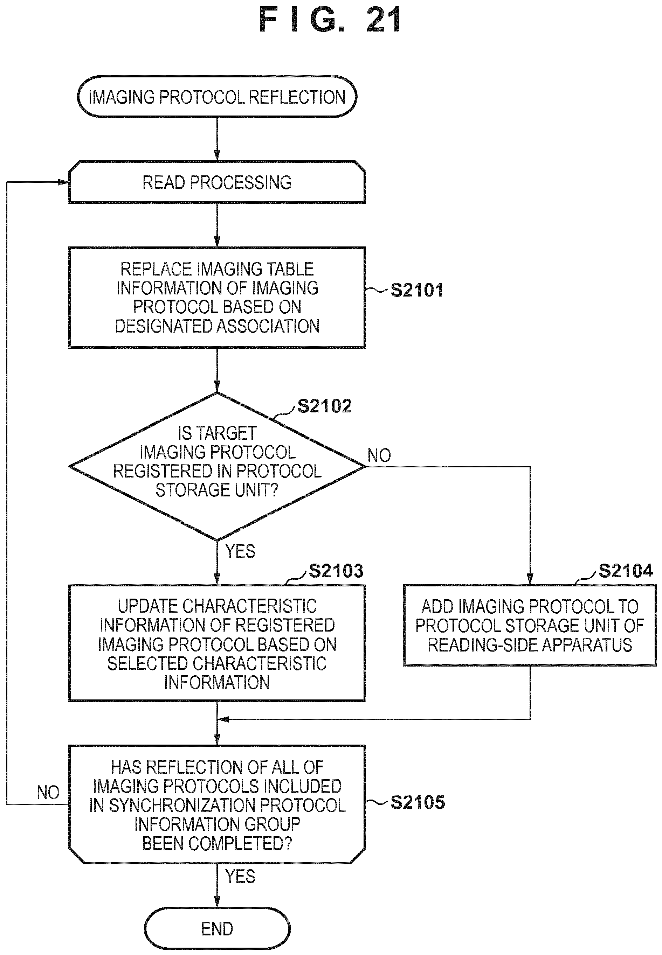

Fig. 21 is a flowchart for explaining the processing procedure of filtering characteristic information to be added and registered (updated) in the reading-side radiation imaging apparatus 102 (self-apparatus) according to the sixth embodiment.

In step S2101, when imaging protocol reflection processing is started, the control unit 202e replaces, based on the association designated in step S503 of Fig. 5, the characteristic information (for example, imaging table information 303) of a processing target imaging protocol based on an imaging protocol obtained from an output-side radiation imaging apparatus 101. As a result, the processing target imaging protocol can be used for imaging by the reading-side radiation imaging apparatus 102.

In step S2102, the control unit 202e determines whether an imaging protocol whose characteristic information (for example, the imaging table information 303) has been replaced is already registered in a protocol storage unit 202c of the reading-side radiation imaging apparatus 102. Whether the imaging protocol is registered can be determined based on the characteristic information included in the imaging protocol. For example, this can be determined by determining whether an imaging protocol matching a protocol name 302a of the processing target imaging protocol and the imaging table information 303 of the characteristic information is present in the protocol storage unit 202c of the reading-side radiation imaging apparatus 102.

In the determination of step S2102, if an imaging protocol whose characteristic information (for example, the imaging table information 303) is already registered in the protocol storage unit 202c of the reading-side radiation imaging apparatus 102 (YES in step S2102), the process advances to step S2103.

Then, in step S2103, the control unit 202e updates the characteristic information based on the characteristic information selected by the update selection unit. For example, if some pieces of the characteristic information are selected, via the shared setting screen (GUI), among the plurality of pieces of characteristic information included in the imaging protocol the control unit 202e, the characteristic information is updated based on some pieces of characteristic information that have been selected. In addition, the control unit 202e holds the characteristic information of each imaging protocol which is already registered in the protocol storage unit 202c, and does not update the characteristic information that has not been selected by the update selection unit. The control unit 202e performs registration (update) processing based on an imaging protocol that has been obtained by excluding identification information 306 from characteristic information 370 (imaging part information 302, the imaging table information 303, image processing information 304, imaging condition information 305, and the identification information 306) included in an imaging protocol 301. Since the identification information is information for identifying an imaging protocol from an RIS and is required to be unique to the radiation imaging system, the control unit 202e performs registration (update) processing by excluding the identification information 306 in order to prevent the coexistence of another piece of identification information with the same contents. The registration (update) processing performed by the control unit 202e can clarify the imaging protocol designation from the RIS.

On the other hand, in the determination of step S2102, if an imaging protocol whose characteristic information (for example, the imaging table information 303) has been replaced is not registered in the protocol storage unit 202c of the reading-side radiation imaging apparatus 102 (NO in step S2102), the process advances to step S2104.

Next, in step S2104, the control unit 202e adds, to the protocol storage unit 202c of the reading-side radiation imaging apparatus 102, the processing target imaging protocol whose characteristic information has been replaced.

In step S2105, the control unit 202e repetitively performs the imaging protocol reflection processing of steps S2101 to S2104 on all of the imaging protocols included in the synchronization protocol information group. Subsequently, this processing ends when the reflection processing related to all of the imaging protocols included in the synchronization protocol information group has been completed.

Note that the processing executed to filter the characteristic information of an imaging protocol included in the synchronization protocol information group is not limited to the identification information 306 described in Fig. 21, and the user may use the GUI to set the piece of the characteristic information 370 that is to be filtered (excluded).

The update selection unit (the display unit 202a, the control unit 202b, and the operation unit 202f) performs display control to cause the display unit 202a to display a shared setting screen (GUI) for selecting the characteristic information of the imaging protocol which is to be updated.

The update selection unit (the display unit 202a, the control unit 202b, and the operation unit 202f) causes the display unit 202a to display, on the shared setting screen (GUI), a reading method setting portion which is used for selecting an overwrite method or a merge method as the reading method (storage method) to be used for storing the imaging protocol in the protocol storage unit 202c.

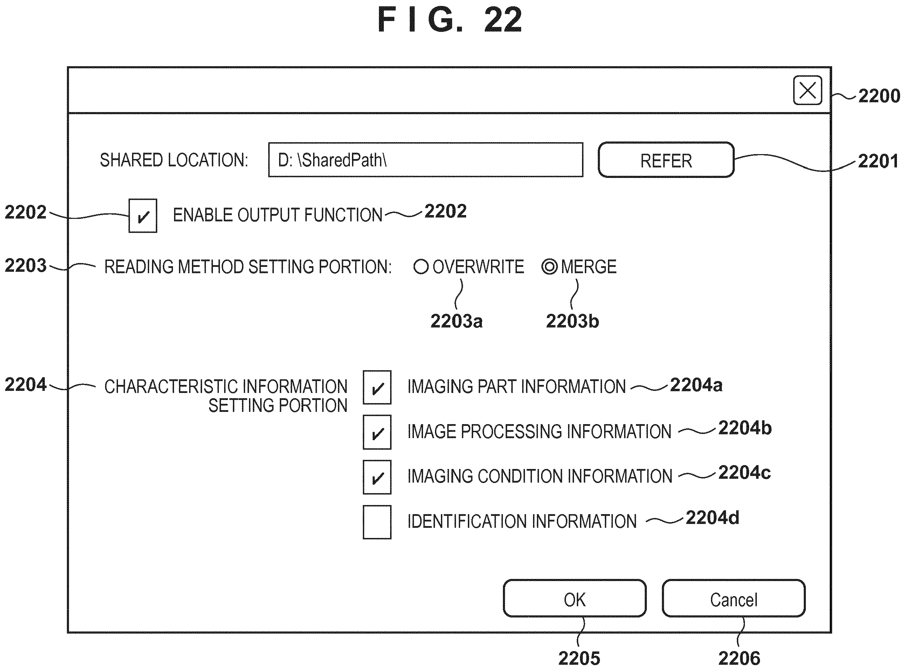

Fig. 22 is a view showing an example of a shared setting screen 2200 (GUI) for setting the characteristic information to be shared. The shared setting screen 2200 is displayed under the display control of the control unit 202b. The shared setting screen 2200 includes a shared location setting portion 2201, an output enable setting portion 2202, a reading method setting portion 2203, and a characteristic information setting portion 2204.

The shared location setting portion 2201 designates a location to store the information to be shared, that is, the storage location (shared location) of the synchronization protocol information group, based on the input from the operation unit 202f. Since the location to which the synchronization protocol information group is output from the output-side radiation imaging apparatus 101 needs to be the same as the location where the synchronization protocol information group is read from the reading-side radiation imaging apparatus 102, this setting will be a setting indicating the same location in all of the radiation imaging apparatuses.

The output enable setting portion 2202 sets, based on the input from the operation unit 202f, a radiation imaging apparatus that has been set with this setting as the output-side radiation imaging apparatus. Since a reading-side radiation imaging apparatus will not be able to identify which radiation imaging apparatus has output the synchronization protocol information group to be read if it is set so that information can be output from all of the radiation imaging apparatuses in a hospital, the control unit 202b sets the radiation imaging apparatus that has been set with this setting as the output-side radiation imaging apparatus 101. On the other hand, a radiation imaging apparatus that has not been set with this setting will function as the reading-side radiation imaging apparatus.

In the reading method setting portion 2203, an overwrite method 2203a or a merge method 2203b can be selectively set as the reading method. The overwrite method 2203a is a reading method in which each imaging protocol that has been uniquely registered to the reading-side radiation imaging apparatus 102 is deleted and replaced by an imaging protocol of a synchronization protocol information group as described in the first to fifth embodiments. In a case in which the overwrite method 2203a is selected based on an input from the operation unit 202f, the control unit 202e deletes all of the imaging protocols stored in the protocol storage unit 202c and stores, in the protocol storage unit 202c, each imaging protocol that has been associated with the characteristic information.

The merge method 2203b is a reading method, described in the sixth and seventh embodiments, in which an imaging protocol that has been uniquely registered to the reading-side radiation imaging apparatus 102 is held while an imaging protocol shared with a synchronization protocol information group is held and an imaging protocol that has not been registered in the reading-side radiation imaging apparatus 102 is added and registered. In a case in which the merge method 2203b is selected based on an input from the operation unit 202f, the control unit 202e holds an imaging protocol that is already registered in the protocol storage unit 202c and updates the characteristic information of the imaging protocol or adds an imaging protocol. In a case in which the merge method is selected, the update selection unit (the display unit 202a, the control unit 202b, and the operation unit 202f) excludes the identification information 306, which includes information for identifying the imaging protocol from an external device 104, as an update target from among the pieces of characteristic information 370 included in the imaging protocol.

In a case in which the reading method setting is the merge method 2203b, the characteristic information setting portion 2204 is used to set whether to prioritize the registration of the characteristic information of a synchronization protocol information group or the characteristic information of a protocol information group which is already registered in the reading-side radiation imaging apparatus 102 (self-apparatus). In the example of Fig. 22, the characteristic information of the processing target is selected when checkbox of the selecting portion to select the characteristic information is checked and is set to the ON setting. The control unit 202e performs control so that a corresponding piece of characteristic information of the synchronization protocol information group will be prioritized and registered with respect to the characteristic information whose checkbox has been checked in the characteristic information setting portion 2204. That is, the control unit 202e will update the imaging protocol registered in the reading-side radiation imaging apparatus 102 by using the characteristic information of the synchronization protocol information group.

More specifically, imaging part information 2204a, image processing information 2204b, and imaging condition information 2204c are updated based on the characteristic information of the imaging protocol included in the synchronization protocol information group. Since the checkbox of the selection portion of identification information 2204d has not been checked and is set to the OFF setting, the identification information 2204d holds the characteristic information characteristic information registered in the reading-side radiation imaging apparatus 102 instead of being updated based on the characteristic information included in the imaging protocol of the synchronization protocol information group.

After the characteristic information setting portion 2204 (reading parameter) has been set, the contents set on the shared setting screen 2200 are stored in the radiation imaging apparatus when a shared setting reflection button 2205 (OK) is pressed. When a shared setting discard button 2206 (Cancel) is pressed, the contents set on the shared setting screen 2200 are discarded.

According to this embodiment, by limiting the characteristic information to be reflected onto the reading-side radiation imaging apparatus 102 (self-apparatus), it becomes possible to hold an imaging protocol that has been uniquely registered to the reading-side radiation imaging apparatus 102 (self-apparatus) while allowing imaging protocols to be shared between the output-side radiation imaging apparatus 101 and the reading-side radiation imaging apparatus 102. By making settings related to imaging protocol sharing for each radiation imaging apparatus, an imaging protocol can be shared by using a method suitable for each radiation imaging apparatus.

### Other Embodiments

Embodiment(s) of the present invention can also be realized by a computer of a system or apparatus that reads out and executes computer executable instructions (e.g., one or more programs) recorded on a storage medium (which may also be referred to more fully as a 'non-transitory computer-readable storage medium') to perform the functions of one or more of the above-described embodiment(s) and/or that includes one or more circuits (e.g., application specific integrated circuit (ASIC)) for performing the functions of one or more of the above-described embodiment(s), and by a method performed by the computer of the system or apparatus by, for example, reading out and executing the computer executable instructions from the storage medium to perform the functions of one or more of the above-described embodiment(s) and/or controlling the one or more circuits to perform the functions of one or more of the above-described embodiment(s). The computer may comprise one or more processors (e.g., central processing unit (CPU), micro processing unit (MPU)) and may include a network of separate computers or separate processors to read out and execute the computer executable instructions. The computer executable instructions may be provided to the computer, for example, from a network or the storage medium. The storage medium may include, for example, one or more of a hard disk, a random-access memory (RAM), a read only memory (ROM), a storage of distributed computing systems, an optical disk (such as a compact disc (CD), digital versatile disc (DVD), or Blu-ray Disc (BD)™), a flash memory device, a memory card, and the like.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.

A radiation imaging system includes a plurality of radiation imaging apparatuses configured to perform radiation imaging. The radiation imaging apparatus comprises: protocol storage means configured to store an imaging protocol which includes characteristic information for controlling the radiation imaging; association means configured to associate characteristic information of another imaging protocol of another radiation imaging apparatus with the characteristic information of the stored imaging protocol; and control means configured to control, based on the association, registration of the characteristic information of the other imaging protocol in the protocol storage means .

## Claims

1. A radiation imaging system that includes a plurality of radiation imaging apparatuses configured to perform radiation imaging, wherein the radiation imaging apparatus comprises
protocol storage means configured to store an imaging protocol which includes characteristic information for controlling the radiation imaging;
association means configured to associate characteristic information of another imaging protocol of another radiation imaging apparatus with the characteristic information of the stored imaging protocol; and
control means configured to control, based on the association, registration of the characteristic information of the other imaging protocol in the protocol storage means.

2. The system according to claim 1, wherein the control means compares, based on the association, the characteristic information of the imaging protocol and the characteristic information of the other imaging protocol and controls the registration based on a result of the comparison.

3. The system according to claim 1, wherein the control means deletes the imaging protocol stored in the protocol storage means and stores, in the protocol storage means, an imaging protocol obtained by replacing the characteristic information of the imaging protocol stored in the protocol storage means with the characteristic information of the other imaging protocol based on the association.

4. The system according to claim 1, further comprising:
data storage means configured to store, in order to perform imaging protocol exchange between the plurality of radiation imaging apparatuses, an imaging protocol stored in the protocol storage means.

5. The system according to claim 4, wherein the control means obtains the imaging protocol stored in the data storage means of the other radiation imaging apparatus as the other imaging protocol of the other radiation imaging apparatus, and
the association means performs the association based on the obtained imaging protocol.

6. The system according to claim 1, wherein the association means is configured to perform display control to cause display means to display an association screen for performing the association.

7. The system according to claim 4, further comprising:
protocol output means configured to output the imaging protocol stored in the protocol storage means,
wherein the protocol output means obtains the imaging protocol stored in the protocol storage means and stores the obtained imaging protocol in the data storage means of the other radiation imaging apparatus.

8. The system according to claim 4, further comprising:
protocol output means configured to output the imaging protocol stored in the protocol storage means,
wherein the protocol output means obtains the imaging protocol stored in the protocol storage means and stores the obtained imaging protocol in a shared server which is connected via a network.

9. The system according to claim 8, wherein the control means obtains the imaging protocol stored in the shared server as the other imaging protocol of the other radiation imaging apparatus, and
the association means performs the association based on the obtained imaging protocol.

10. The system according to claim 3, further comprising:
registration selection means configured to select information to be registered in the protocol storage means based on a setting,
wherein the registration selection means stores, in the protocol storage means, an imaging protocol selected by the registration selection means from the other imaging protocols associated by the association means.

11. The system according to claim 10, wherein the registration selection means performs display control to cause display means to display an imaging protocol selection screen for setting the information to be registered in the protocol storage means.

12. The system according to claim 4, further comprising:
output selection means configured to select, from the imaging protocols stored in the protocol storage means, an imaging protocol to be output outside based on a setting,
wherein the output selection means selects, from the imaging protocols obtained from the protocol storage means, the imaging protocol to be output outside based on the setting and stores the selected imaging protocol in the data storage means.

13. The system according to claim 12, wherein the output selection means performs display control to cause display means to display an imaging protocol selection screen for setting the imaging protocol to be output outside.

14. The system according to claim 1, wherein in a case in which the characteristic information of the imaging protocol and the characteristic information of the other imaging protocol match, the control means replaces the characteristic information of the stored imaging protocol with the characteristic information of the other imaging protocol and registers the replaced characteristic information in the protocol storage means.

15. The system according to claim 1, wherein the control means holds the imaging protocol stored in the protocol storage means, and stores, in the protocol storage means, an imaging protocol obtained by replacing the characteristic information of the imaging protocol stored in the protocol storage means with the characteristic information of the other imaging protocol based on the association.

16. The system according to claim 1, wherein the characteristic information includes at least one piece of imaging part information of a subject who is to undergo the radiation imaging, imaging table information which includes information of a detector to be used in the radiation imaging, image processing information to be applied to an image captured by the radiation imaging, imaging condition information for controlling the detector or the radiation imaging apparatus, and identification information for identifying an imaging protocol from an external device.

17. The system according to claim 16, wherein in a case in which information included in the imaging table information of the imaging protocol and information included in the imaging table information of the other imaging protocol match, the control means replaces the characteristic information of the stored imaging protocol with the characteristic information of the other imaging protocol and registers the replaced characteristic information in the protocol storage means.

18. The system according to claim 1, wherein the association means associates first characteristic information of the other imaging protocol of the other radiation imaging apparatus with first characteristic information of the stored imaging protocol, and
the control means controls, based on the association, registration of second characteristic information of the other imaging protocol in the protocol storage means.

19. A radiation imaging method of a radiation imaging system that includes a plurality of radiation imaging apparatuses each including protocol storage means configured to store an imaging protocol which includes characteristic information for controlling radiation imaging, the method comprising:
associating characteristic information of another imaging protocol of another radiation imaging apparatus with the characteristic information of the stored imaging protocol; and
controlling, based on the association, registration of the characteristic information of the other imaging protocol in the protocol storage means.

20. A radiation imaging apparatus that performs radiation imaging of a subject, comprising:
protocol storage means configured to store an imaging protocol which includes characteristic information for controlling the radiation imaging;
association means configured to associate characteristic information of another imaging protocol of another radiation imaging apparatus with the characteristic information of the stored imaging protocol; and
control means configured to control, based on the association, registration of the characteristic information of the other imaging protocol in the protocol storage means.

21. A storage medium storing a program configured to cause a computer to function as each means of a radiation imaging system defined in claim 1.
